# EUROPÄISCHE PATENTSCHRIFT

(11) **EP 1 610 701 B1**
(45) Veröffentlichungstag und Bekanntmachung des Hinweises auf die Patenterteilung: **31.10.2007**
(21) Anmeldenummer: 03816422.4
(22) Anmeldetag: 28.03.2003
(51) Int. Cl.: A61B 17/86, A61B 17/72

(54) **VERRIEGELUNGSSCHRAUBE**
LOCKING SCREW
VIS DE VERROUILLAGE

(43) Veröffentlichungstag der Anmeldung: 04.01.2006
(73) Patentinhaber: Synthes GmbH, 4436 Oberdorf (CH)
(72) Erfinder: SCHLIENGER, André, CH-4053 Basel (CH); SENN, Peter, CH-4437 Waldenburg (CH); BUETTLER, Markus, CH-4702 Oensingen (CH)
(74) Vertreter: Lusuardi, Werther
(86) Internationale Anmeldenummer: PCT/CH2003/000202
(87) Internationale Veröffentlichungsnummer: WO 2004/084745

(56) Entgegenhaltungen:
- EP-A- 0 554 915
- EP-A- 0 845 245
- WO-A-02/089683
- FR-A- 2 704 140

## Beschreibung

Die Erfindung bezieht sich auf eine Verriegelungsschraube gemäss dem Oberbegriff des Patentanspruchs 1.

Die Verriegelung von Marknägeln gehört zum Stand der Technik. Die Einführung der Verriegelungsschrauben in die Querbohrungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - relativ zur Querbohrung ein gewisses Spiel auf.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Zusammen mit der Flexibilität des Materials und der Gesamtvorrichtung kann dies kumuliert eine Grösse annehmen, welche eine erfolgreiche Heilung verhindert, oder massgeblich verzögert. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube bewegen kann.

Eine beispielhafte Schraube ist in der EP-A-0 554 915 gezeigt.

Hier will die Erfindung Abhilfe schaffen. Der Erfindung liegt die Aufgabe zugrunde, eine Verriegelungsschraube zu schaffen, mit der das vorhandene Spiel zwischen ihr und der Querbohrung in einem Verriegelungs-Marknagel eliminiert werden kann.

Die Erfindung löst die gestellte Aufgabe mit einer Verriegelungsschraube, welche die Merkmale des Anspruchs 1 aufweist.

Als Zentrallinie wird dabei die Verbindungslinie der Schwerpunkte der axial aufeinanderfolgenden, orthogonalen Querschnittsflächen der Verriegelungsschraube angesehen.

Die durch die Erfindung erreichten Vorteile sind im wesentlichen darin zu sehen, dass dank der erfindungsgemässen Verriegelungsschraube das Spiel zwischen der Querbohrung des Marknagels und Verriegelungsschraube eliminiert werden kann. Weitere Vorteile sind folgende:
- die Einbringgenauigkeit und der Zeitaufwand für den Chirurgen bleiben im bisherigen Rahmen;
- die Festigkeit der Verriegelungsschraube beliebt erhalten; und
- die Extraktion bei einem allfälligen Schraubenbruch ist gewährleistet.

Die Erfindung und Weiterbildungen der Erfindung werden im folgenden anhand der teilweise schematischen Darstellungen mehrerer Ausführungsbeispiele noch näher erläutert.

Bei einer besonderen Ausführungsform der Erfindung besitzt die Verriegelungsschraube keine Rotations-Symmetrieachse.

Die Zentrallinie kann in einer oder mehreren Ebene liegen. Bei einer besonderen Ausführungsform wird die Zentrallinie aus mehreren gegeneinander verschobenen Geraden gebildet, wodurch sich eine einfachere Herstellung ergibt.

Eine zwischen den beiden Austrittspunkte der Zentrallinie verlaufende Verbindungsgerade weist bei einer besonderen Ausführungsform mindestens an einer zwischen den beiden Austrittspunkten liegenden Stelle einen Abstand x von der Zentrallinie auf, wobei x > 0,01 mm, vorzugsweise x > 0,10 mm ist. Der Abstand x gehorcht zweckmässigerweise der Bedingung 0,01 d < x < 0,30 d, vorzugsweise der Bedingung 0,05 d < x < 0,20 d.

Bei einer weiteren Ausführungsform ist die Verriegelungsschraube zwischen den beiden Austrittspunkten der Zentrallinie, mit dem Abstand L zueinander, in drei Abschnitte A, B und C unterteilt, wobei sich
A) Abschnitt A vom Austrittspunkt am Schraubenkopf um den Betrag 0,10 L bis 0,25 L gegen den Austrittspunkt am freien Ende des Schraubenschaftes hin erstreckt;
B) Abschnitt B vom Austrittspunkt am freien Ende des Schraubenschaftes um den Betrag 0,10 bis 0,25 L gegen den Austrittspunkt am Schraubenkopf hin erstreckt; wobei
C) Abschnitt C zwischen den beiden Abschnitten A und B angeordnet ist und die Länge C = (L - A - B) aufweist; und
D) die Zentrallinie in den Abschnitten A und B im wesentlichen geradlinig und koaxial zueinander verläuft.

Dadurch ergibt sich der Vorteil, dass die Verriegelung in der gegenüberliegenden Kortikalis durch Rotationsbewegung um die Verbindungsgerade und die Verriegelung in der kopfseitigen Kortikalis t möglichst bohrachsengerecht erfolgt.

Bei einer weiteren Ausführungsform ist die Zentrallinie vorzugsweise nur im Abschnitt C S-förmig gekrümmt oder exzentrisch. Die Zentrallinie kann - vorzugsweise nur im Abschnitt C - einen Wendepunkt aufweisen. Sie kann auch - vorzugsweise nur im Abschnitt C - mindestens zwei Wendepunkte im Abstand y voneinander aufweisen.

Die Verriegelungsschraube kann zusammen mit einem Verriegelungsmarknagel verwendet werden, welcher mindestens eine Querbohrung aufweist. Zweckmässigerweise weist die Querbohrung ein Querschnittsprofil P mit einer maximalen Ausdehnung a in Richtung der Zentrallinie gemessen und einer maximalen Ausdehnung b senkrecht zu a gemessen auf, wobei einerseits a > b und anderseits a > d < b gilt.

Das Querschnittsprofil kann kreisförmig sein, mit a = b. Zweckmässigerweise gilt im weiteren die Bedingung 0,70 b < d < 0,95 b, vorzugsweise 0,8 b < d < 0,9 b.

Der Abstand x gehorcht zweckmässigerweise der Bedingung x < (b - d + 1 mm), wobei b der Durchmesser der Querbohrung in mm und d der Durchmesser des Schraubenschaftes in mm ist.

Bei einer weiteren Ausführungsform gehorcht der Abstand x der Bedingung 0,05 (b - d) < x < 0,35 (b - d), vorzugsweise der Bedingung 1,5 (b - d) < x < 2,2 (b - d), wobei d der Durchmesser des Schraubenschaftes in mm ist und b der Durchmesser der Querbohrung in mm ist.

Bei einer weiteren Ausführungsform gehorcht der Abstand y zwischen zwei benachbarten Wendepunkten im wesentlichen der Bedingung D = n y, wobei n eine ungerade Zahl ist und D der Durchmesser des Marknagels ist.

Es zeigen:
Fig. 1 eine perspektivische Ansicht einer Verriegelungsschraube;
Fig. 2 einen Längsschnitt durch die Verriegelungsschraube nach Fig. 1;
Fig. 3 einen Längsschnitt durch einen Verriegelungsmarknagel mit einer Querbohrung, in welcher die Verriegelungschraube nach Fig. 1 eingeführt ist; und
Fig. 4 einen Längsschnitt durch eine modifizierte Verriegelungsschraube.

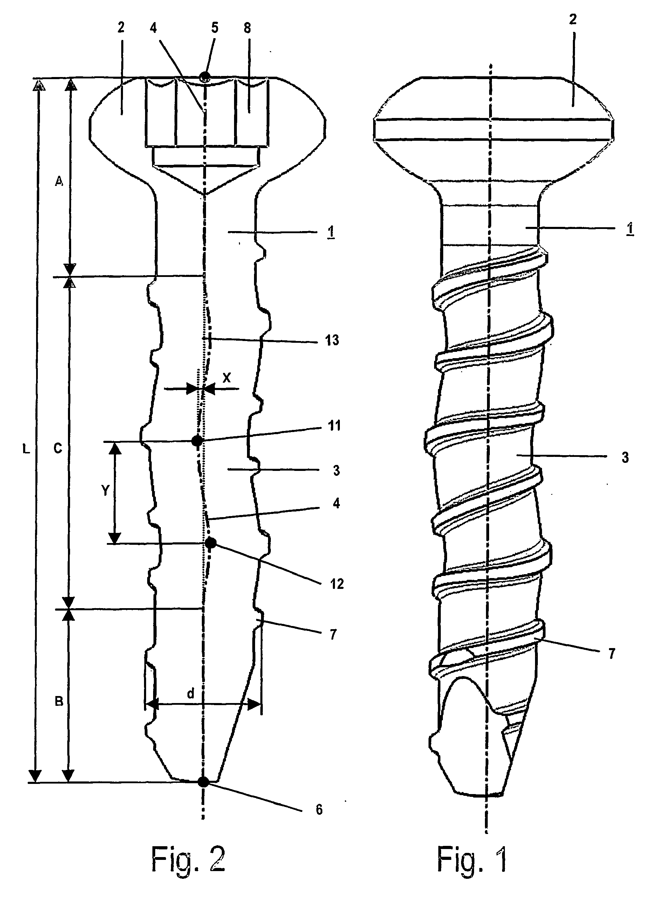

Die in den Fig. 1 und 2 gezeigte Verriegelungsschraube umfasst einen Schraubenkopf 2 mit einem Innensechskant 8, einem Schraubenschaft 3 mit einem Aussengewinde 7 und einer Zentrallinie 4, welche einen Austrittspunkt 5 am Schraubenkopf 2 und einen Austrittspunkt 6 am freien Ende des Schraubenschaftes 3 aufweist. Die Zentrallinie 4 ist keine Gerade wie bei den üblichen geraden Schrauben, sondern besteht im gezeigten Ausführungsbeispiel nach Fig. 2 (im weiter unten definierten Längenbereich C) aus einer Schlangenlinie, die in der Zeichenebene liegt und zwei Wendepunkte 11,12 besitzt.

Die durch die beiden Austrittspunkte 5,6 der Zentrallinie 4 verlaufende Verbindungsgerade 13 weicht deshalb stellenweise von der Zentrallinie 4 um den variablen Betrag x ab. Im gezeigten Ausführungsbeispiel beträgt die maximale Abweichung von x - in den Wendepunkten der Zentrallinie gemessen - 0,2 mm.

Zwischen den beiden Austrittspunkten 5,6 der Zentrallinie 4, welche den Abstand L zueinander aufweisen, ist die Zentrallinie 4 in drei Abschnitte A, B und C unterteilt. Abschnitt A erstreckt sich vom Austrittspunkt 5 am Schraubenkopf 2 um den Betrag 1/6 L gegen den Austrittspunkt 6 am freien Ende des Schraubenschaftes 3 hin und verläuft im wesentlichen geradlinig. Abschnitt B erstreckt sich vom Austrittspunkt 6 am freien Ende des Schraubenschaftes 3 um den Betrag 1/6 L gegen den Austrittspunkt 5 am Schraubenkopf 2 hin und verläuft ebenfalls im wesentlichen geradlinig sowie koaxial zum Abschnitt A. Abschnitt C ist zwischen den beiden Abschnitten A und B angeordnet und weist wie oben beschrieben eine Krümmung auf.

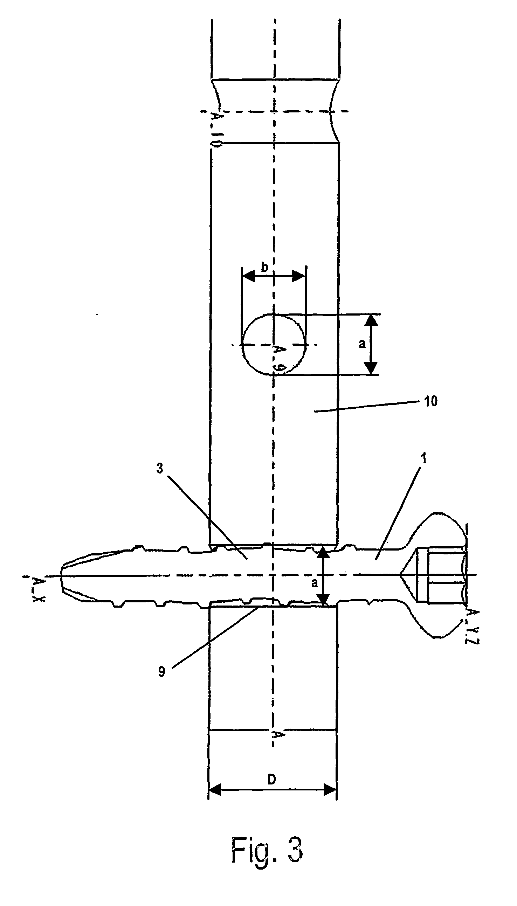

In Fig. 3 ist gezeigt wie die Verriegelungsschraube 1 in der Querbohrung 9 eines Marknagels 10 eingeführt ist. Der Schraubenschaft 3 weist dabei einen Durchmesser d (Fig. 2) auf, der kleiner ist als das Mass "a" der Querbohrung 9.

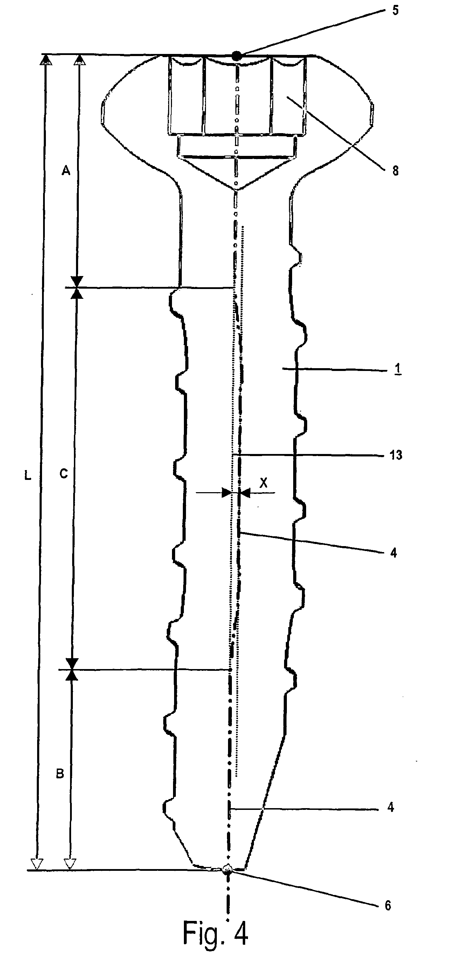

In Fig. 4 ist eine Modifikation der Verriegelungsschraube 1 dargestellt, bei welcher die Zentrallinie 4 aus drei gegeneinander versetzten Geraden besteht. Der Abstand x zwischen der Zentrallinie 4 und der Verbindungsgeraden im Abschnitt C zwischen den beiden Austrittspunkten 5 und 6 beträgt bei diesem Ausführungsbeispiel 0,15 mm.

Im Folgenden wird noch kurz die Technik des Eindrehens der Verriegelungsschraube in die Querbohrung eines Marknagels erläutert:
a) der Chirurg dreht die Verriegelungsschraube 1 standardmässig durch die Querbohrung 9 des Marknagels 10 ein;
b) die relativ dünne und weiche Kortikalis gibt beim Hindurchwinden der Verriegelungsschraube 1 nach, so dass über die Kortikalisdicke keine Verspannung erfolgt;
c) im Bereich des Marknagels 10 streckt sich die Verriegelungsschraube 1 etwas, wegen der Reaktion der Querbohrungswandung, so dass eine erhöhte Eindrehkraft sowie eine erhöhte Haltekraft resultiert;
d) im Falle einer Kannulierung des Marknagels 10 windet sich die Verriegelungsschraube 1 durch die Eintrittsöffnung der Querbohrung 9 des Marknagels 10 in die Querbohrung 9, da der Durchmesser D des Marknagels 10 grösser ist als der Abstand y zwischen den beiden Wendepunkten 11,12. Die Verriegelungsschraube 1 wird durch das Eindrehmoment, resp. durch die Vorschubkraft des Chirurgen spätestens beim Fassen in der Gegenkortikalis in eine elastisch Deformation gezwungen, was zur winkelstabilen Verriegelung des Marknagels führt.

## Patentansprüche

1. Verriegelungsschraube (1) mit einem Schraubenkopf (2), einem Schraubenschaft (3) mit dem Durchmesser d, welcher aus einem Kern (14) und einem Aussengewinde (7) besteht, und einer Zentrallinie (4), welche als Verbindungslinie der Schwerpunkte der axial aufeinanderfolgenden, orthogonalen Querschnittsflächen des Kerns (14) definiert ist und welche einen Austrittspunkt (5) am Schraubenkopf (2) und einen Austrittspunkt (6) am freien Ende des Schraubenschaftes (3) aufweist,
**dadurch gekennzeichnet, dass**
die Zentrallinie (4) nicht durchgehend geradlinig ist und einen Wendepunkt (11) aufweist.

2. Verriegelungsschraube nach Anspruch 1, **dadurch gekennzeichnet, dass** sie keine Rotations-Symmetrieachse besitzt.

3. Verriegelungsschraube nach Anspruch 1 oder 2, **dadurch gekennzeichnet, dass** die Zentrallinie (4) in einer Ebene liegt.

4. Verriegelungsschraube nach Anspruch 1 oder 2, **dadurch gekennzeichnet, dass** die Zentrallinie (4) in mehreren Ebenen liegt.

5. Verriegelungsschraube nach einem der Ansprüche 1 bis 4, **dadurch gekennzeichnet, dass** die Zentrallinie (4) aus mehreren gegeneinander verschobenen Geraden gebildet ist.

6. Verriegelungsschraube nach einem der Ansprüche 1 bis 5, **dadurch gekennzeichnet, dass** eine durch die beiden Austrittspunkte (5,6) der Zentrallinie (4) verlaufende Verbindungsgerade (13) mindestens an einer zwischen den beiden Austrittspunkten (5,6) liegenden Stelle einen Abstand x von der Zentrallinie (4) aufweist, wobei x > 0,01 mm, vorzugsweise x > 0,10 mm ist.

7. Verriegelungsschraube nach einem der Ansprüche 1 bis 6, **dadurch gekennzeichnet, dass** der Abstand x der Bedingung 0,01 d < x < 0,30 d; vorzugsweise der Bedingung 0,05 d < x < 0,20 d gehorcht.

8. Verriegelungsschraube nach einem der Ansprüche 1 bis 7, **dadurch gekennzeichnet**, sie zwischen den beiden Austrittspunkten (5;6) der Zentrallinie (4), mit dem Abstand L zueinander, in drei Abschnitte A, B und C unterteilt ist, wobei sich
A) Abschnitt A vom Austrittspunkt (5) am Schraubenkopf (2) um den Betrag 0,10 L bis 0,25 L gegen den Austrittspunkt (6) am freien Ende des Schraubenschaftes (3) hin erstreckt;
B) Abschnitt B vom Austrittspunkt (6) am freien Ende des Schraubenschaftes (3) um den Betrag 0,10 bis 0,25 L gegen den Austrittspunkt (5) am Schraubenkopf (2) hin erstreckt; wobei
C) Abschnitt C zwischen den beiden Abschnitten A und B angeordnet ist und die Länge C = (L - A - B) aufweist; und
D) die Zentrallinie (4) in den Abschnitten A und B im wesentlichen geradlinig und koaxial zueinander verläuft.

9. Verriegelungsschraube nach Anspruch 8, **dadurch gekennzeichnet, dass** die Zentrallinie (4) vorzugsweise nur im Abschnitt C S-förmig gekrümmt oder exzentrisch ist.

10. Verriegelungsschraube nach Anspruch 8 oder 9, **dadurch gekennzeichnet, dass** die Zentrallinie (4) vorzugsweise, nur im Abschnitt C einen Wendepunkt (11) aufweist.

11. Verriegelungsschraube nach einem der Ansprüche 8 bis 10, **dadurch gekennzeichnet, dass** die Zentrallinie (4) vorzugsweise nur im Abschnitt C mindestens zwei Wendepunkte (11;12) im Abstand y voneinander aufweist.

12. Verriegelungsschraube nach einem der Ansprüche 1 bis 11, **dadurch gekennzeichnet, dass** der Durchmesser "d" des Schraubenschaftes im wesentlichen konstant ist.

13. Verriegelungsschraube nach einem der Ansprüche 1 bis 12, **dadurch gekennzeichnet, dass** der Durchmesser des Kerns (14) im wesentlichen konstant ist.

14. Verriegelungsmarknagel (10) mit einer Verriegelungsschraube (1) nach einem der Ansprüche 1 bis 13, **dadurch gekennzeichnet, dass** der Verriegelungsmarknagel (10) mindestens eine Querbohrung (9) aufweist mit ein Querschnittsprofil P, einer maximalen Ausdehnung a in Richtung der Zentrallinie (4) gemessen und einer maximalen Ausdehnung b senkrecht zu a gemessen aufweist, wobei einerseits a > b und anderseits a > d < b gilt.

15. Verriegelungsmarknagel (10) nach Anspruch 14, **dadurch gekennzeichnet, dass** das Querschnittsprofil P kreisförmig mit a = b ist und im weiteren die Bedingung 0,70 b < d < 0,95 b, vorzugsweise 0,8 b < d < 0,9 b gilt.

16. Verriegelungsmarknagel (10) nach Anspruch 14 oder 15, **dadurch gekennzeichnet, dass** der Abstand x der Bedingung x < (b - d + 1 mm) gehorcht, wobei b der Durchmesser der Querbohrung (9) in mm und d der Durchmesser des Schraubenschaftes (3) in mm ist.

17. Verriegelungsmarknagel (10) nach einem der Ansprüche 14 bis 16, **dadurch gekennzeichnet, dass** der Abstand x der Bedingung 0,05 (b - d) < x < 0,35 (b - d), vorzugsweise der Bedingung 1,5 (b - d) < x < 2,2 (b - d) gehorcht, wobei d der Durchmesser des Schraubenschaftes (3) in mm ist.

18. Verriegelungsmarknagel (10) nach einem der Ansprüche 14 bis 17, **dadurch gekennzeichnet, dass** der Abstand y zwischen zwei benachbarten Wendepunkten (11;12) im wesentlichen der Bedingung D = n y gehorcht, wobei n eine ungerade Zahl ist und D der Durchmesser des Marknagels ist.

## Claims

1. Locking screw (1) with a screw head (2), a screw shaft (3) with the diameter d, which consists of a core (14) and an external thread (7), and with a central line (4), which is defined as the line connecting the centers of gravity of the axially sequential, orthogonal cross-sectional surfaces of the core (14) and which has an exit point (5) at the screw head (2) and an exit point (6) at the free end of the screw shaft (3),
**characterized in that**
the central line (4) is not a continuously straight line and has a point of inflection (11).

2. The locking screw of claim 1, **characterized in that** it does not have a rotational axis of symmetry.

3. The locking screw of claims 1 or 2, **characterized in that** the central line (4) lies in a plane.

4. The locking screw of claims 1 or 2, **characterized in that** the central line (4) lies in several planes.

5. The locking screw of one of the claims 1 to 4, **characterized in that** the central line (4) is formed from several mutually transposed straight lines.

6. The locking screw of one of the claims 1 to 5, **characterized in that** a straight connecting line (13), passing through the two exit points (5, 6) of the central line (4), is at a distance x from the central line (4) at least at one place between the two exit points (5, 6), with x > 0,01 mm and preferably with x > 0,10 mm.

7. The locking screw of one of the claims 1 to 6, **characterized in that** the distance x fulfills the condition that 0,01 d < x < 0,30 d and preferably the condition that 0,05 d < x < 0,20 d.

8. The locking screw of one of the claims 1 to 7, **characterized in that** it is divided between the two exit points (5; 6) of the central line (4), between which there is a distance L, into three sections A, B and C,
A) section A extending from the exit point (5) at the screw head (2) by the amount of 0,10 L and 0,25 L towards the exit point (6) at the free end of the screw shaft (3),
B) section B extending from the exit point (6) at the free end of the screw shaft (3) by the amount of 0,10 to 0,25 L towards the exit point (5) at the screw head (2),
C) section C being disposed between the two sections A and B and having a length C = (L-A-B) and
D) the central line (4) in sections A and B being essentially linear and extendsing coaxially to one another.

9. The locking screw of claim 8, **characterized in that** the central line (4) is curved S-shaped or is eccentric only in section C.

10. The locking screw of claims 8 or 9, **characterized in that** the central line (4) has a point of inflection (11) preferably only in section C.

11. The locking screw of one of the claims 8 to 10, **characterized in that** central line (4) has at least two points of inflection (11; 12) at a distance y from one another preferably only in section C.

12. The locking screw of one of the claims 1 to 11, **characterized in** at the diameter "d" of the screw shaft is essentially constant.

13. The locking screw of one of the claims 1 to 12, **characterized in that** the diameter of the core (14) is essentially constant.

14. The locking medullary pin (10) with a locking screw (1) of one of the claims 1 to 13, **characterized in that** the locking medullary pin (10) has at least one transverse borehole (9) with a cross-sectional profile P, a maximum extent "a" measured in the direction of central line (4) and a maximum extent "b" measured perpendicularly to "a", on the one hand, a > b and, on the other, a > d < b.

15. The locking medullary pin (10) of claim 14, **characterized in that** the cross-sectional profile is circular with a = b and, moreover, the condition that 0,70 b < d < 0,95 b and preferably 0,8 b < d < 0,9 b applies.

16. The locking medullary pin of claims 14 or 15, **characterized in that** the distance x fulfills the condition x < (b-d+1 mm), b being the diameter of the transverse borehole (9) in mm and d being the diameter of the screw shaft (3) in mm.

17. The locking medullary pin of one of the claims 14 to 16, **characterized in that** the distance x fulfills the condition 0,05 (b-d) < x < 0,35 (b-d) and preferably the condition 1,5 (b-d) < x < 2,2 (b-d), d being the diameter of the screw shaft (3) in mm.

18. The locking medullary pin of one of the claims 14 to 17, **characterized in that** the distance y between two adjacent points of inflection (11; 12) essentially fulfills the condition D = ny, n being an odd number and D the diameter of the medullary pin.

## Revendications

1. Vis de verrouillage (1) comprenant une tête de vis (2), une tige de vis (3) de diamètre d, qui se compose d'un noyau (14) et d'un filetage (7), et une ligne centrale (4), qui est définie comme ligne de liaison des centres de gravité des surfaces de section transversale orthogonales du noyau (14) qui se succèdent axialement et qui présente un point de sortie (5) au niveau de la tête de vis (2) et un point de sortie (6) au niveau de l'extrémité libre de la tige de vis (3),
**caractérisée en ce que**
la ligne centrale (4) n'est pas droite de bout en bout et présente un point d'inflexion (11).

2. Vis de verrouillage selon la revendication 1, **caractérisée en ce qu'**elle ne possède aucun axe de symétrie de rotation.

3. Vis de verrouillage selon la revendication 1 ou 2, **caractérisée en ce que** la ligne centrale (4) est située sur un plan.

4. Vis de verrouillage selon la revendication 1 ou 2, **caractérisée en ce que** la ligne centrale (4) est située sur plusieurs plans.

5. Vis de verrouillage selon l'une quelconque des revendications 1 à 4, **caractérisée en ce que** la ligne centrale (4) est formée de plusieurs droites qui sont décalées les unes par rapport aux autres.

6. Vis de verrouillage selon l'une quelconque des revendications 1 à 5, **caractérisée en ce qu'**une droite de liaison (13) qui passe par les deux points de sortie (5, 6) de la ligne centrale (4) présente, à au moins un endroit situé entre les deux points de sortie (5, 6), une distance x par rapport à la ligne centrale (4), avec x > 0,01 mm, de préférence x > 0,10 mm.

7. Vis de verrouillage selon l'une quelconque des revendications 1 à 6, **caractérisée en ce que** la distance x remplit la condition 0,01 d < x < 0,30 d, de préférence la condition 0,05 d < x < 0,20 d.

8. Vis de verrouillage selon l'une quelconque des revendications 1 à 7, **caractérisée en ce qu'**elle est divisée en trois segments A, B et C entre les deux points de sortie (5 ; 6) de la ligne centrale (4), avec une distance L entre eux, dans laquelle
A) le segment A s'étend depuis le point de sortie (5) situé au niveau de la tête de vis (2) sur une distance de 0,10 L à 0,25 L vers le point de sortie (6) situé au niveau de l'extrémité libre de la tige de vis (3) ;
B) le segment B s'étend depuis le point de sortie (6) situé au niveau de l'extrémité libre de la tige de vis (3) sur une distance de 0,10 L à 0,25 L vers le point de sortie (5) situé au niveau de la tête de vis (2) ; où
C) le segment C est situé entre les deux segments A et B et présente la longueur C = (L - A - B) ; et
D) la ligne centrale (4) traverse les segments A et B de manière essentiellement droite et coaxiale.

9. Vis de verrouillage selon la revendication 8, **caractérisée en ce que** la ligne centrale (4) est courbée en forme de S ou est excentrique de préférence uniquement dans le segment C.

10. Vis de verrouillage selon la revendication 8 ou 9, **caractérisée en ce que** la ligne centrale (4) présente un point d'inflexion (11) de préférence uniquement dans le segment C.

11. Vis de verrouillage selon l'une quelconque des revendications 8 à 10, **caractérisée en ce que** la ligne centrale (4) présente au moins deux points d'inflexion (11 ; 12) séparés par une distance y de préférence uniquement dans le segment C.

12. Vis de verrouillage selon l'une quelconque des revendications 1 à 11, **caractérisée en ce que** le diamètre "d" de la tige de vis est essentiellement constant.

13. Vis de verrouillage selon l'une quelconque des revendications 1 à 12, **caractérisée en ce que** le diamètre du noyau (14) est essentiellement constant.

14. Clou intramédullaire à verrouillage (10) avec une vis de verrouillage (1) selon l'une quelconque des revendications 1 à 13, **caractérisée en ce que** le clou intramédullaire à verrouillage (10) présente au moins un alésage transversal (9) ayant un profil de section transversale P, une extension maximale a mesurée en direction de la ligne centrale (4) et une extension maximale b mesurée perpendiculairement à a, avec d'une part a > b et d'autre part a > d < b.

15. Clou intramédullaire à verrouillage (10) selon la revendication 14, **caractérisé en ce que** le profil de section transversale P est circulaire avec a = b et, en outre, la condition 0,70 b < d < 0,95 b, de préférence 0,8 b < d < 0,9 b, s'applique.

16. Clou intramédullaire à verrouillage (10) selon la revendication 14 ou 15, **caractérisé en ce que** la distance x remplit la condition x < (b - d + 1 mm), où b est le diamètre de l'alésage transversal (9) en mm et d est le diamètre de la tige de vis (3) en mm.

17. Clou intramédullaire à verrouillage (10) selon l'une quelconque des revendications 14 à 16, **caractérisé en ce que** la distance x remplit la condition 0,05 (b - d) < x < 0,35 (b - d), de préférence la condition 1,5 (b - d) < x < 2,2 (b - d), où d est le diamètre de la tige de vis (3) en mm.

18. Clou intramédullaire à verrouillage (10) selon l'une quelconque des revendications 14 à 17, **caractérisé en ce que** la distance y entre deux points d'inflexion consécutifs (11 ; 12) remplit essentiellement la condition D = n y, où n est un nombre impair et D est le diamètre du clou intramédullaire.
